# EUROPEAN PATENT APPLICATION

(11) **EP 4 420 681 A1**
(43) Date of publication of application: **28.08.2024**
(21) Application number: 22882856.2
(22) Date of filing: 18.10.2022
(51) Int. Cl.: A61K 47/54, A61K 47/64, A61K 47/65, A61K 31/40, A61P 35/00, A61P 37/02

(54) **CONJUGATE DRUG PREPARATION, PREPARATION METHOD THEREFOR AND USE THEREOF**

(30) Priority: 19.10.2021 CN 202111215700
(71) Applicant: Coherent Biopharma (Suzhou), Limited, Industrial Park Suzhou Jiangsu 215123 (CN)
(72) Inventor: FAN, Sixiang, Suzhou, Jiangsu 215123 (CN); SHANG, Peipei, Suzhou, Jiangsu 215123 (CN); WU, Fan, Suzhou, Jiangsu 215123 (CN)
(74) Representative: Perani & Partners S.p.A.
(86) International application number: PCT/CN2022/125935
(87) International publication number: WO 2023/066251

(57) **Abstract**

A conjugate pharmaceutical preparation, a preparation method therefor and use thereof in the field of biomedicines. The preparation contains a ligand-drug conjugate, a pH regulator, and a freeze-dried excipient. pH screening is carried out on the preparation containing the ligand-drug conjugate, the pH regulator and the freeze-dried excipient used by the preparation are defined, and at the same time, the dosing sequence and dispensing environment during drug preparation are investigated, parameters of the freeze-drying process are screened, and finally the effects that the characters, the moisture content, the impurity content, the pH value and the like of the preparation in the storage period can be kept stable are finally achieved.

## Description

### Priority and Related Application

The present application claims the right of priority for Chinese patent application no. 202111215700.9, filed with the China National Intellectual Property Administration on October 19, 2021 and entitled "CONJUGATE PHARMACEUTICAL PREPARATION, PREPARATION METHOD THEREFOR AND USE THEREOF", which is incorporated herein by reference in its entirety.

### Technical Field

The present invention relates to the field of biomedicines. In particular, the present invention relates to a conjugate pharmaceutical preparation, in particular to a pharmaceutical preparation comprising a ligand-drug conjugate, and a preparation method therefor and the use thereof.

### Background Art

In recent years, although tumor chemotherapy has been considerably advanced, treatment of solid tumors, which most seriously harm human life and health and account for more than 90% of malignant tumors, has failed to achieve satisfactory results. Pharmacologists and oncologists in the industry are becoming increasingly aware of: to improve the efficacy of tumor treatment, a breakthrough progress can be made by starting from the mechanism of the occurrence and development of tumors.

Studies on the molecular mechanism involved in the occurrence and development of tumors have shown that in tumor cells, the regulation of various fundamental processes in cells are out of control. Therefore, researchers have turned their attention to specific molecular and biological targets that play a role in the pathological process of cancers. There are a number of specific or overexpressed receptors on the surfaces of tumor cells and diseased cells. Ligands of these receptors can be divided into proteins, polypeptides, and small molecules. These ligands have the characteristics of good specificity, moderate affinity, and obvious biological effect when binding to receptors and can obviously improve the targeting property and efficacy of the drugs while reducing the toxicity when coupling with therapeutic drugs to form ligand-drug conjugates (LDCs).

The ligand-drug conjugate has endocytosis mediation function, and can realize the combination of targeting and endocytosis structures. The affinity and targeting property of the drug conjugate to diseased cells are enhanced utilizing a dual-targeting ligand, such that an efficient toxin drug such as monomethyl auristatin E (MMAE) can be carried. Linkers make the conjugate unable to release drug molecules outside cells (intercellular substances, blood circulation system, etc.), thereby ensuring the stability of the drug in the circulation *in vivo,* reducing the drug toxicity and not generating toxic effects on normal cells. After entering targeted cells, the linker is cleaved to release the drug molecules with a therapeutic effect, thereby avoiding the generation of multiple drug resistance (MDR).

LDC preparations need to ensure that they maintain good stability during preparation, during long-term storage and in the subsequent use period. The stability of the LDC in the preparation depends on the pH regulator, stabilizer, surfactant, *etc.,* used in the preparation. If the LDC is not properly formulated in a liquid, the LDC in the liquid solution tends to decompose, aggregate, or undergo undesirable chemical modifications, *etc.* There is a need in the art for novel pharmaceutical preparations comprising LDC that is sufficiently stable and suitable for administration to a subject. Therefore, suitable LDC preparations need to be prepared for the treatment or prevention of diseases.

### Summary of the Invention

### Problems to be solved by the invention

With regard to the above-mentioned need in the art for novel pharmaceutical preparations comprising LDC that is stable and suitable for administration to a subject, the present invention is intended to provide a conjugate pharmaceutical preparation, in particular to a pharmaceutical preparation comprising a ligand-drug conjugate, a preparation method therefor and the use thereof. By limiting pH value, the type and amount of a pH regulator, a freeze-dried excipient, the dosing sequence and dispensing environment during drug preparation, and parameters of a freeze-drying process, the need of keeping good stability in the storage period is met.

### Solutions to solve problems

[1]. A pharmaceutical preparation, comprising an active drug and optionally a pharmaceutically acceptable adjuvant.
[2]. The pharmaceutical preparation according to [1], wherein the active drug comprises a ligand-drug conjugate or a pharmaceutically acceptable salt thereof; the pharmaceutically acceptable adjuvant comprises one or more of a freeze-dried excipient and a pH regulator.
[3]. The pharmaceutical preparation according to [1] or [2], wherein the active drug comprises a ligand-drug conjugate or a pharmaceutically acceptable salt thereof; the pharmaceutically acceptable adjuvant comprises one or more of a freeze-dried excipient, a pH regulator, and a solvent.
[4]. The pharmaceutical preparation according to [3], wherein the solvent is water; preferably, the solvent is purified water; more preferably, the purified water is sterile water, distilled water, or deionized water; and more preferably, the sterile water is sterile water for injection, single-distilled water, or double-distilled water.
[5]. The pharmaceutical preparation according to [3], wherein the active drug has a concentration of 2 mg/mL to 8 mg/mL, such as 2.0 mg/mL, 2.2 mg/mL, 2.4 mg/mL, 2.6 mg/mL, 2.8 mg/mL, 3.0 mg/mL, 3.2 mg/mL, 3.4 mg/mL, 3.6 mg/mL, 3.8 mg/mL, 4.0 mg/mL, 4.2 mg/mL, 4.4 mg/mL, 4.6 mg/mL, 4.8 mg/mL, 5.0 mg/mL, 5.2 mg/mL, 5.4 mg/mL, 5.6 mg/mL, 5.8 mg/mL, 6.0 mg/mL, 6.2 mg/mL, 6.4 mg/mL, 6.6 mg/mL, 6.8 mg/mL, 7.0 mg/mL, 7.2 mg/mL, 7.4 mg/mL, 7.6 mg/mL, 7.8 mg/mL, or 8.0 mg/mL.
[6]. The pharmaceutical preparation according to [2] or [3], wherein the pH regulator comprises a buffer salt.
[7]. The pharmaceutical preparation according to [6], wherein the pH regulator further comprises an acidic substance and/or an alkaline substance.
[8]. The pharmaceutical preparation according to [6], wherein the buffer salt comprises one or more of an acetate, a phosphate, a citrate, or a hydrate thereof; preferably, each 1 molecule of the hydrate comprises 0.5, 1, 1.5, 2.0, 2.5 or 3 molecules of water; and preferably, the buffer salt is one or more of sodium acetate, anhydrous sodium citrate, sodium citrate dihydrate or sodium dihydrogen phosphate.
[9]. The pharmaceutical preparation according to [7], wherein the acidic substance comprises an acid; preferably, the acidic substance is one or more of hydrochloric acid, acetic acid, or citric acid; the alkaline substance comprises an alkali or a salt; and preferably, the alkaline substance is one or more of sodium hydroxide, sodium carbonate or sodium bicarbonate.
[10]. The pharmaceutical preparation according to any one of [6] to [9], wherein when the buffer salt is selected from anhydrous sodium citrate or sodium citrate dihydrate, the buffer salt has a concentration of 2.85 mg/mL to 11.4 mg/mL, such as 2.85 mg/mL, 3.5 mg/mL, 4.2 mg/mL, 5.7 mg/mL, 6.5 mg/mL, 7.6 mg/mL, 8.5 mg/mL, 9.8 mg/mL, 10.5 mg/mL, or 11.4 mg/mL.
[11]. The pharmaceutical preparation according to [2] or [3], wherein the freeze-dried excipient comprises a polyol, such as one or more of mannitol and xylitol; preferably, the freeze-dried excipient is mannitol, preferably the freeze-dried excipient has a concentration of 20 mg/mL to 150 mg/mL, such as 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, or 150 mg/mL; and more preferably the freeze-dried excipient has a concentration of 40 mg/mL to 60 mg/mL.
[12]. The pharmaceutical preparation according to [11], wherein the freeze-dried excipient further comprises a saccharide, such as one or more of a monosaccharide, a disaccharide, and a polysaccharide; preferably, a mass ratio of the saccharide to the polyol is 1:1 to 1:5, such as 1:1, 1:1.5, 1:2, 1:2.5, 1:3, 1:3.5, 1:4, 1:4.5, 1:5; and more preferably, a mass ratio of the saccharide to the polyol is 1:2 to 1:4.
[13]. The pharmaceutical preparation according to [12], wherein the monosaccharide comprises one or more of glucose and fructose; the disaccharide comprises one or more of sucrose, trehalose, and maltose; and the polysaccharide comprises one or more of cyclodextrin and dextran.
[14]. The pharmaceutical preparation according to any one of [11] to [13], wherein the freeze-dried excipient is mannitol and sucrose; and preferably, a mass ratio of the sucrose to the mannitol is 1:4.
[15]. The pharmaceutical preparation according to any one of [3] to [14], wherein the pharmaceutical preparation has a pH value of 5.0 to 8.0; such as 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.
[16]. The pharmaceutical preparation according to [1], wherein the pharmaceutical preparation comprises a ligand-drug conjugate or a pharmaceutically acceptable salt thereof, a freeze-dried excipient, a pH regulator, and a solvent; wherein the pharmaceutical preparation has a pH value of 5.0 to 8.0; preferably, each 1 mL of the pharmaceutical preparation comprises 5 mg of a dual ligand-drug conjugate or a pharmaceutically acceptable salt thereof, 50 mg of mannitol, 5.7 mg of sodium citrate dihydrate, optionally an acidic substance or alkaline substance, and the balance made up of water; wherein the pharmaceutical preparation has a pH value of 5.0 to 8.0; preferably, each 1 mL of the pharmaceutical preparation comprises 5 mg of a dual ligand-drug conjugate or a pharmaceutically acceptable salt thereof, 40 mg of mannitol, 10 mg of sucrose, 4.5 mg of anhydrous sodium citrate, optionally an acidic substance or alkaline substance, and the balance made up of water; wherein the pharmaceutical preparation has a pH value of 5.0 to 8.0; preferably, each 1 mL of the pharmaceutical preparation comprises 4 mg of a dual ligand-drug conjugate or a pharmaceutically acceptable salt thereof, 50 mg of mannitol, an appropriate amount of sodium acetate, optionally an acidic substance or alkaline substance, and the balance made up of water; wherein the pharmaceutical preparation has a pH value of 5.0 to 8.0; preferably, each 1 mL of the pharmaceutical preparation comprises 6 mg of a dual ligand-drug conjugate or a pharmaceutically acceptable salt thereof, 50 mg of mannitol, an appropriate amount of sodium dihydrogen phosphate, optionally an acidic substance or alkaline substance, and the balance made up of water; and wherein the pharmaceutical preparation has a pH value of 5.0 to 8.0.
[17]. The pharmaceutical preparation according to any one of [2] to [16], wherein the ligand-drug conjugate is a dual ligand-drug conjugate; preferably, the dual ligand-drug conjugate is a drug conjugate targeting a PSMA receptor and a TRPV6 receptor; preferably, the dual ligand-drug conjugate has the following structure: 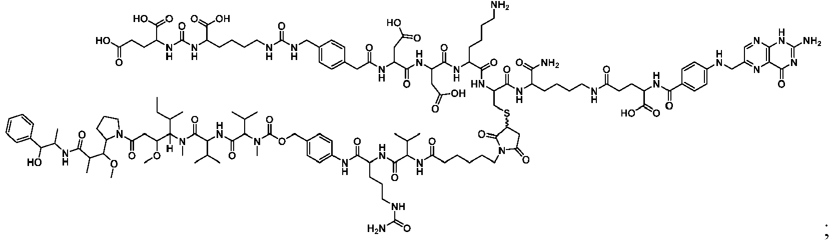 and more preferably, the dual ligand-drug conjugate has the following structure: 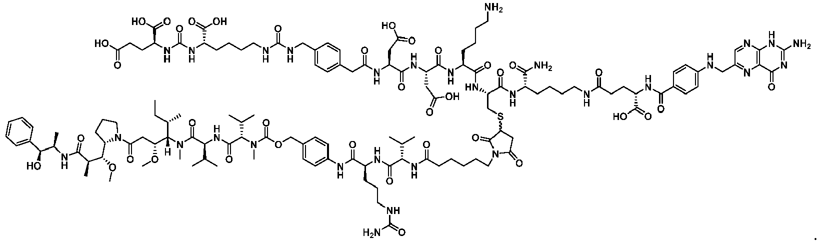
[18]. The pharmaceutical preparation according to any one of [1] to [17], wherein the pharmaceutical preparation is sterile.
[19]. The pharmaceutical preparation according to any one of [1] to [17], wherein the pharmaceutical preparation is stable during freezing and thawing.
[20]. A preparation method for preparing the pharmaceutical preparation according to any one of [1] to [19], comprising the following steps: using a prescribed amount of an active drug and optionally adding a pharmaceutically acceptable adjuvant, and carrying out uniform mixing to obtain the pharmaceutical preparation.
[21]. The preparation method according to [20], wherein the method comprises the following steps: uisng a prescribed amount of a ligand-drug conjugate or a pharmaceutically acceptable salt thereof, a freeze-dried excipient, a pH regulator, and a solvent, dissolving each component in the solvent at once or in batches, optionally regulating the pH value to 5.0 to 8.0 using an acidic substance or an alkaline substance in the pH regulator, and carrying out uniform mixing to obtain the pharmaceutical preparation; preferably, when dissolving each component in the solvent, dissolving the pH regulator in the solvent at the same time or prior to the ligand-drug conjugate or the pharmaceutically acceptable salt thereof.
[22]. The preparation method according to [20] or [21], wherein the preparation method is optionally carried out in a dark place; and preferably, the preparation method is carried out in a dark place.
[23]. The preparation method according to any one of [20] to [22], wherein the preparation method is carried out at a temperature of 15°C to 40°C.
[24]. The preparation method according to any one of [20] to [23], wherein the preparation method is optionally carried out under nitrogen protection; and preferably, the preparation method is not carried out under nitrogen protection.
[25]. A freeze-dried preparation, wherein the freeze-dried preparation is prepared by freeze-drying the pharmaceutical preparation according to any one of [1] to [19].
[26]. A method for preparing the freeze-dried preparation according to [25], comprising the following steps: (i) freezing the pharmaceutical preparation at -45°C; (ii) carrying out first drying on the pharmaceutical preparation at -15°C to -5°C; and (iii) carrying out second drying on the pharmaceutical preparation at 15°C to 25°C; for example, the temperature of the first drying is -15°C, -10°C, or -5°C; the temperature of the second drying is 15°C or 25°C; preferably, the preparation method comprises the following steps: (i) freezing the pharmaceutical preparation at -45°C; (ii) carrying out first drying on the pharmaceutical preparation at -15°C; and (iii) carrying out second drying on the pharmaceutical preparation at 25°C.
[27]. The freeze-dried preparation according to [25], wherein the freeze-dried preparation is stable at room temperature.
[28]. A liquid preparation, reconstituted by the freeze-dried preparation according to [25] using water.
[29]. The liquid preparation according to [28], wherein the water comprises one or more of distilled water, pure water, and sterile water; and preferably, the freeze-dried preparation has a pH value of 5.0 to 8.0 after being reconstituted using water.
[30]. A preparation method for preparing the liquid preparation according to [28] or [29], comprising the following steps: mixing the freeze-dried preparation with sterile water to obtain the liquid preparation.
[31]. A drug-containing delivery device, comprising one of the following preparations: the pharmaceutical preparation according to any one of [1] to [19], the freeze-dried preparation according to [25], or the liquid preparation according to [28] or [29].
[32]. A pre-filled syringe, comprising one of the following preparations: the pharmaceutical preparation according to any one of [1] to [19], the freeze-dried preparation according to [25] or the liquid preparation according to [28] or [29], preferably for use in intravenous injection or intramuscular injection.
[33]. Use of the pharmaceutical preparation according to any one of [1] to [19], the freeze-dried preparation according to [25], or the liquid preparation according to [28] or [29] in the preparation of a delivery device or a pre-filled syringe or a drug for enhancing an immune effector cell response and/or reducing immunosuppression in a subject.
[34]. Use of the pharmaceutical preparation according to any one of [1] to [19], the freeze-dried preparation according to [25], or the liquid preparation according to [28] or [29] in the preparation of a delivery device or a pre-filled syringe or a drug for the treatment and/or prevention of a cancer, an immune disease, a cardiovascular disease, a metabolic disease and a neurological disease in a subject.
[35]. The use according to [34], wherein the cancer comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, testicular cancer, skin cancer, lymphoma, and multiple myeloma; the immune disease comprises one or more of a connective tissue disease, systemic sclerosis, rheumatoid arthritis, and systemic lupus erythematosus; the cardiovascular disease comprises one or more of angina pectoris, myocardial infarction, apoplexy, heart attack, a hypertensive heart disease, a rheumatic heart disease, cardiomyopathy, cardiac arrhythmia, and a congenital heart disease; the metabolic disease comprises one or more of diabetes mellitus, gout, obesity, hypoglycemia, hyperglycemia, and dyslipidemia; the neurological disease comprises one or more of Alzheimer's disease, Parkinson's disease, Huntington's disease, head injury, multiple sclerosis, vertigo, coma, and epilepsy.

### Effects of the Invention

In the present invention, pH screening is carried out on the preparation containing the ligand-drug conjugate, the pH regulator and the freeze-dried excipient used by the preparation are defined, and at the same time, the dosing sequence and dispensing environment during drug preparation are investigated, parameters of the freeze-drying process are screened, and finally the effects that the characters, the moisture content, the impurity content, the pH value and the like of the preparation in the storage period can be kept stable are finally achieved.

### Brief Description of the Drawings

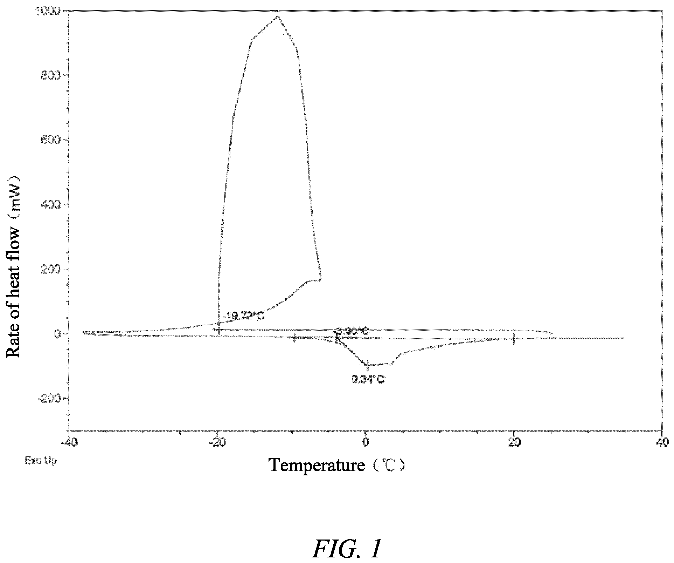

FIG. 1 is a DSC curve graph of a drug prescription solution containing CR19213.

### Detailed Description of Embodiments

Embodiments of the present invention are described below, but the present invention is not limited thereto. The present invention is not limited to each composition described below. Various modifications can be made within the scope of protection of the present invention. Embodiments and examples obtained by appropriately combining the technical means disclosed in different embodiments and examples are also included in the technical scope of the present invention. In addition, all documents recited in the present description are incorporated herein by reference.

Unless otherwise stated, instrument devices, reagents, materials, *etc.* used in the present invention can be acquired by conventional commercial means.

Unless otherwise defined, technical and scientific terms used in the present invention have the same meaning as commonly understood by those of ordinary skill in the technical field to which the present invention belongs.

In the present description, reference to "some particular/preferred embodiments", "other particular/preferred embodiments", "some particular/preferred technical solutions", "other particular/preferred technical solutions" and the like mean that a particular element (e.g., feature, structure, property, and/or characteristic) described in connection with the embodiment is included in at least one embodiment described herein, and may or may not be present in other embodiments. In addition, it should be understood that the element can be combined in any suitable manner in various embodiments.

In the present description, a numerical range represented by "numerical value A to numerical value B" or "numerical value A-numerical value B" means a range including endpoint values A and B.

In the present description, where "%" appears, it means mass or weight percentage, *i.e.,* "% by mass" or "% by weight", unless otherwise specifically stated.

In the present description, the meaning represented by "may" includes both the meaning of carrying out certain treatment and the meaning of not carrying out certain treatment. In the present description, the "optional" or "optionally" means that the subsequently described event or circumstance may or may not occur, and that the description includes instances where the event occurs and instances where it does not.

In the present description, the "room temperature" or "normal temperature" means an ambient temperature of about 25°C.

The term "comprise" and any variations thereof in the description and claims of the present invention and the accompanying drawings described above are intended to cover non-exclusive inclusion.

In the present invention, the "pharmaceutically acceptable" means those that are, within the scope of reasonable medical judgment, suitable for use in contact with cells of humans and other animals without undue toxicity, irritation, allergic response and the like, and are commensurate with a reasonable benefit/risk ratio.

In the present invention, the "pharmaceutically acceptable adjuvant" refers to excipients and additives used in drug manufacturing and prescription formulating, such as a pH regulator; and the "pharmaceutically acceptable adjuvant" is a substance which is other than an active ingredient or a precursor, has been reasonably assessed in terms of safety and is contained in a pharmaceutical preparation. In addition to acting as an excipient, regulating the pH value, acting as a carrier, and improving stability, the pharmaceutical adjuvant has important functions such as solubilization, hydrotropy, release control, and is an important ingredient that may affect the quality, safety and effectiveness of a drug.

In the present invention, the "pharmaceutically acceptable salt" refers to a relatively non-toxic, inorganic and organic acid addition salt and alkali addition salt of the conjugate compound of the present application. Representative acid addition salts include hydrobromides, hydrochlorides, sulfates, bisulfates, phosphates, nitrates, acetates, oxalates, valerates, oleates, palmitates, stearates, laurates, borates, benzoates, lactates, tosylates, citrates, maleates, fumarates, succinates, tartrates, naphthoates, mesylates, glucoheptonates, lactiobionates, sulphamates, malonates, salicylates, propionates, methylene-bis-b-hydroxynaphthoates, gentisates, isethionates, di-p-toluoyltartrates, ethanesulphonates, benzenesulphonates, p-toluenesulphonates, cyclohexylsulphamates, quinateslaurylsulphonate, *etc.* Alkali addition salts include pharmaceutically acceptable metal and amine salts. Suitable metal salts include sodium, potassium, calcium, barium, zinc, magnesium, and aluminum salts. In some embodiments, sodium and potassium salts are preferred. Suitable inorganic alkali addition salts are prepared from base of metal which include, for example, sodium hydride, sodium hydroxide, potassium hydroxide, calcium hydroxide, aluminum hydroxide, lithium hydroxide, magnesium hydroxide, and zinc hydroxide. Suitable amine base addition salts are prepared from sufficiently basic amines to form stable salts, and preferably include the following common amines in pharmaceutical chemistry due to their low toxicity and acceptability for medical use: ammonia, ethylenediamine, N-methylglucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, tetramethylammonium hydroxide, triethylamine, dibenzylamine, diphenylhydroxylmethylamine, dehydroabietylamine, N-ethylpiperidine, benzylamine, tetramethylammonium, tetraethylammonium, methylamine, dimethylamine, trimethylamine, ethylamine, alkaline amino acids (e.g., lysine and arginine), dicyclohexylamine, *etc.*

In the present invention, the "ligand-drug conjugate (LDC)" is a product in which a biologically active drug is linked to a ligand via a chemical linker, wherein the ligand acts as a carrier to transport a small molecule drug to a cell of interest in a targeted manner, the ligand may be a polypeptide or a small molecule, and the biologically active drug may be monomethyl auristatin E (MMAE). Preferably, the ligand-drug conjugate is a dual ligand-drug conjugate, preferably, the dual ligand-drug conjugate is a drug conjugate targeting a PSMA receptor and a TRPV6 receptor. More preferably, the ligand-drug conjugate of the present invention is dual ligand conjugate CR19213, the ligands target a PSMA receptor and a TRPV6 receptor respectively, and the loaded drug is MMAE, and the structure of the ligand-drug conjugate is as follows: 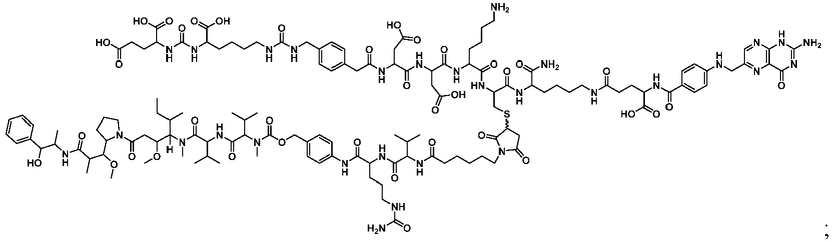 further preferably, the dual ligand-drug conjugate has the following structure:

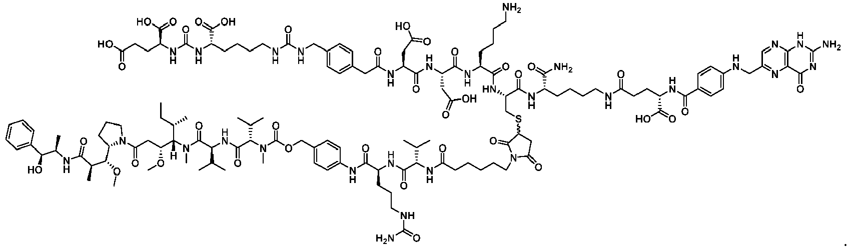

In the present invention, the "pH regulator" is a reagent capable of maintaining the pH value of a solution in an acceptable range, and mainly acids, alkalis, and salts having a buffering effect, such as common hydrochloric acid, acetic acid, sodium hydroxide, sodium dihydrogen phosphate, and sodium acetate. In some embodiments, the pH regulator used in the preparation of the present invention may control the pH value of the preparation of the present invention in a pH range of about 5.0 to 8.0. In some specific embodiments, the preparation of the present invention has a pH value of about 5.0, 5.5, 6.0, 6.5, 7.0, and 8.0.

In the present invention, the "polyol" refers to a broad class of alcohols containing two or more hydroxyl groups in the molecule, and for example, can be mannitol, inositol, ethylene glycol, polyethylene glycol, *etc.*

In the present invention, the "reconstitution" refers to dissolving and/or suspending a solid preparation (e.g., a freeze-dried preparation) in a physiologically acceptable solution.

In the present invention, the "about" means that the defined numerical value is approximate and that the particular numerical value in the relevant data needs not be very precise.

In the present invention, the "freeze-dried preparation" means a sterile solid for injection prepared by a freeze-drying method.

In the present invention, the "freeze-dried excipient", also known as a freeze-dried protectant, refers to an additive added during freeze-drying and storage of foods, drugs and organisms in order to keep the stability and activity of the product under the influence of many factors (such as chemical component, freezing rate, freezing and dehydration stress, glass transition temperature, residual moisture in the dried solid, and temperature and humidity of the storage environment). For example, the freeze-dried excipient may be a polyol, a saccharide, an amino acid, an inorganic salt, a protein, *etc.,* such as mannitol, ethylene glycol, sucrose, sodium glutamate, sodium acetate, calcium carbonate, and bovine serum albumin.

In the present invention, the "stability (stable)" in the "stability of preparation" or "stable preparation" means that the moisture content, appearance, pH value, related substances, content, insoluble particles, character after re-dissolution, *etc.* of a product remain substantially unchanged, do not obviously change, or change within a pharmaceutically acceptable range during the preparation and storage, and that the quality of the preparation may be controlled within at least 24 months.

In the present invention, the "DSC" refers to differential scanning calorimetry, a thermal analysis method. The input power difference between a sample and a reference is measured as a function of temperature under programmed temperature control. The curve recorded by differential scanning calorimeter is called DSC curve, which takes the endothermic or exothermic rate of a sample, namely the rate of heat flow as the ordinate and takes temperature T or time t as the abscissa and can measure a variety of thermodynamic and kinetic parameters. The method has a wide temperature range, high resolution, and less sample amount and is suitable for analysis of inorganic substances, organic compounds and drugs.

### [Pharmaceutical preparation comprising CR19213 and preparation method therefor]

The present invention provides a pharmaceutical preparation, which may comprise an active drug and optionally a pharmaceutically acceptable adjuvant.

In one embodiment, the above-mentioned pharmaceutical preparation may comprise a ligand-drug conjugate, a freeze-dried excipient, and a pH regulator.

In one embodiment, the above-mentioned pharmaceutical preparation may comprise a ligand-drug conjugate, a freeze-dried excipient, a pH regulator, and a solvent.

In one embodiment, the ligand-drug conjugate in the above-mentioned pharmaceutical preparation may be a dual ligand-drug conjugate, targeting a PSMA receptor and a TRPV6 receptor.

In one embodiment, the solvent in the above-mentioned pharmaceutical preparation is water.

In one preferred embodiment, the solvent in the above-mentioned pharmaceutical preparation is purified water.

In another preferred embodiment, the solvent in the above-mentioned pharmaceutical preparation is sterile water, distilled water, or deionized water.

In one more preferred embodiment, the solvent in the above-mentioned pharmaceutical preparation is sterile water for injection, single-distilled water, or double-distilled water.

In one embodiment, the active drug in the above-mentioned pharmaceutical preparation has a concentration of 2 mg/mL to 8 mg/mL, such as 2.0 mg/mL, 2.2 mg/mL, 2.4 mg/mL, 2.6 mg/mL, 2.8 mg/mL, 3.0 mg/mL, 3.2 mg/mL, 3.4 mg/mL, 3.6 mg/mL, 3.8 mg/mL, 4.0 mg/mL, 4.2 mg/mL, 4.4 mg/mL, 4.6 mg/mL, 4.8 mg/mL, 5.0 mg/mL, 5.2 mg/mL, 5.4 mg/mL, 5.6 mg/mL, 5.8 mg/mL, 6.0 mg/mL, 6.2 mg/mL, 6.4 mg/mL, 6.6 mg/mL, 6.8 mg/mL, 7.0 mg/mL, 7.2 mg/mL, 7.4 mg/mL, 7.6 mg/mL, 7.8 mg/mL, or 8.0 mg/mL.

In one embodiment, the pH regulator in the above-mentioned pharmaceutical preparation may comprise a buffer salt.

Further, the buffer salt comprised in the pH regulator in the above-mentioned pharmaceutical preparation may be selected from one or more of an acetate, a phosphate, a citrate, or a hydrate thereof.

Further, each 1 molecule of the buffer salt hydrate in the above-mentioned pharmaceutical preparation comprises 0.5, 1, 1.5, 2.0, 2.5 or 3 molecules of water.

In one preferred embodiment, the pH regulator in the above-mentioned pharmaceutical preparation may comprise sodium acetate.

In another preferred embodiment, the pH regulator in the above-mentioned pharmaceutical preparation may comprise sodium dihydrogen phosphate.

In still another preferred embodiment, the pH regulator in the above-mentioned pharmaceutical preparation may comprise anhydrous sodium citrate.

In still another preferred embodiment, the pH regulator in the above-mentioned pharmaceutical preparation may comprise sodium citrate dihydrate.

In one embodiment, when the buffer salt comprised in the pH regulator in the above-mentioned pharmaceutical preparation is selected from anhydrous sodium citrate or sodium citrate dihydrate, the buffer salt has a concentration of 2.85 mg/mL to 11.4 mg/mL, such as 2.85 mg/mL, 5.7 mg/mL, or 11.4 mg/mL.

In one embodiment, the pH regulator in the above-mentioned pharmaceutical preparation may comprises a buffer salt and an acidic substance and/or an alkaline substance.

Further, the buffer salt comprised in the pH regulator in the above-mentioned pharmaceutical preparation may be selected from one or more of an acetate, a phosphate, a citrate, or a hydrate thereof; the acidic substance may be selected from an acid, such as one or more of hydrochloric acid, acetic acid, or citric acid; the alkaline substance may be selected from an alkali or a salt, such as one or more of sodium hydroxide, sodium carbonate, or sodium bicarbonate.

In one preferred embodiment, the pH regulator in the above-mentioned pharmaceutical preparation may comprise sodium acetate and hydrochloric acid or sodium hydroxide.

In another preferred embodiment, the pH regulator in the above-mentioned pharmaceutical preparation may comprise sodium dihydrogen phosphate and hydrochloric acid or sodium hydroxide.

In still another preferred embodiment, the pH regulator in the above-mentioned pharmaceutical preparation may comprise anhydrous sodium citrate and hydrochloric acid or sodium hydroxide, wherein the anhydrous sodium citrate has a concentration of 2.85 mg/mL to 11.4 mg/mL, such as 2.85 mg/mL, 3.5 mg/mL, 4.2 mg/mL, 5.7 mg/mL, 6.5 mg/mL, 7.6 mg/mL, 8.5 mg/mL, 9.8 mg/mL, 10.5 mg/mL, or 11.4 mg/mL.

In still another preferred embodiment, the pH regulator in the above-mentioned pharmaceutical preparation may comprise sodium citrate dihydrate and hydrochloric acid or sodium hydroxide, wherein the sodium citrate dihydrate has a concentration of 2.85 mg/mL to 11.4 mg/mL, such as 2.85 mg/mL, 3.5 mg/mL, 4.2 mg/mL, 5.7 mg/mL, 6.5 mg/mL, 7.6 mg/mL, 8.5 mg/mL, 9.8 mg/mL, 10.5 mg/mL, or 11.4 mg/mL.

In one embodiment, the freeze-dried excipient in the above-mentioned pharmaceutical preparation may comprise a polyol, such as one or more of mannitol and xylitol.

In one preferred embodiment, the freeze-dried excipient in the above-mentioned pharmaceutical preparation comprises mannitol, preferably, the mannitol has a concentration of 20 mg/mL to 150 mg/mL, such as 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, or 150 mg/mL; and more preferably the mannitol has a concentration of 40 mg/mL to 60 mg/mL.

In one embodiment, the freeze-dried excipient in the above-mentioned pharmaceutical preparation comprises a polyol and a saccharide, wherein the saccharide is one or more of a monosaccharide, a disaccharide, and a polysaccharide, preferably, a mass ratio of the saccharide to the polyol is 1:1 to 1:5, such as 1:1, 1:1.5, 1:2, 1:2.5, 1:3, 1:3.5, 1:4, 1:4.5, or 1:5; and preferably, a mass ratio of the saccharide to the polyol is 1:2 to 1:4.

Further, the monosaccharide in the freeze-dried excipient in the above-mentioned pharmaceutical preparation comprises one or more of glucose and fructose; the disaccharide comprises one or more of sucrose, trehalose, and maltose; the polysaccharide comprises one or more of cyclodextrin and dextran.

In one preferred embodiment, the freeze-dried excipient in the above-mentioned pharmaceutical preparation may comprise mannitol and sucrose, preferably, a mass ratio of the sucrose to the mannitol is 1:4.

In one embodiment, the above-mentioned pharmaceutical preparation may have a pH value of 5.0 to 8.0, such as 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

In one preferred embodiment, the above-mentioned pharmaceutical preparation may have a pH value of 5.5 to 6.5, such as 5.5, 5.6, 5.7, 5.8, 5.9, 6.0, 6.1, 6.2, 6.3, 6.4, or 6.5.

In one embodiment, the above-mentioned pharmaceutical preparation comprises a ligand-drug conjugate or a pharmaceutically acceptable salt thereof, a freeze-dried excipient, a pH regulator, and a solvent; and wherein the pharmaceutical preparation has a pH value of 5.0 to 8.0.

In one preferred embodiment, each 1 mL of the above-mentioned pharmaceutical preparation comprises 5 mg of a dual ligand-drug conjugate or a pharmaceutically acceptable salt thereof, 50 mg of mannitol, 5.7 mg of sodium citrate dihydrate, optionally an acidic substance or alkaline substance, and the balance made up of water; and wherein the pharmaceutical preparation has a pH value of 5.0 to 8.0.

In one preferred embodiment, each 1 mL of the above-mentioned pharmaceutical preparation comprises 5 mg of a dual ligand-drug conjugate or a pharmaceutically acceptable salt thereof, 40 mg of mannitol, 10 mg of sucrose, 4.5 mg of anhydrous sodium citrate, optionally an acidic substance or alkaline substance, and the balance made up of water; and wherein the pharmaceutical preparation has a pH value of 5.0 to 8.0.

In one preferred embodiment, each 1 mL of the pharmaceutical preparation comprises 4 mg of a dual ligand-drug conjugate or a pharmaceutically acceptable salt thereof, 50 mg of mannitol, an appropriate amount of sodium acetate, optionally an acidic substance or alkaline substance, and the balance made up of water; and wherein the pharmaceutical preparation has a pH value of 5.0 to 8.0.

In one preferred embodiment, each 1 mL of the pharmaceutical preparation comprises 6 mg of a dual ligand-drug conjugate or a pharmaceutically acceptable salt thereof, 50 mg of mannitol, an appropriate amount of sodium dihydrogen phosphate, optionally an acidic substance or alkaline substance, and the balance made up of water; and wherein the pharmaceutical preparation has a pH value of 5.0 to 8.0.

In one embodiment, the above-mentioned pharmaceutical preparation is sterile.

In one embodiment, the above-mentioned pharmaceutical preparation is stable during freezing and thawing.

In one embodiment, a preparation method for preparing the above-mentioned pharmaceutical preparation comprises the following steps: using a prescribed amount of an active drug and optionally adding a pharmaceutically acceptable adjuvant, and carrying out uniform mixing to obtain the pharmaceutical preparation.

In one preferred embodiment, a preparation method for preparing the above-mentioned pharmaceutical preparation comprises the following steps: uisng a prescribed amount of a ligand-drug conjugate or a pharmaceutically acceptable salt thereof, a freeze-dried excipient, a pH regulator, and a solvent, dissolving each component in the solvent at once, and carrying out uniform mixing to obtain the pharmaceutical preparation; preferably, when dissolving each component in the solvent, dissolving the pH regulator in the solvent at the same time or prior to the ligand-drug conjugate or the pharmaceutically acceptable salt thereof.

In one preferred embodiment, a preparation method for preparing the above-mentioned pharmaceutical preparation comprises the following steps: uisng a prescribed amount of a ligand-drug conjugate or a pharmaceutically acceptable salt thereof, a freeze-dried excipient, a pH regulator, and a solvent, dissolving each component in the solvent in batches, and carrying out uniform mixing to obtain the pharmaceutical preparation; preferably, when dissolving each component in the solvent, dissolving the pH regulator in the solvent at the same time or prior to the ligand-drug conjugate or the pharmaceutically acceptable salt thereof.

In another preferred embodiment, a preparation method for preparing the above-mentioned pharmaceutical preparation comprises the following steps: uisng a prescribed amount of a ligand-drug conjugate or a pharmaceutically acceptable salt thereof, a freeze-dried excipient, a pH regulator, and a solvent, dissolving each component in the solvent at once, regulating the pH value to 5.0 to 8.0 using an acidic substance or an alkaline substance in the pH regulator, and carrying out uniform mixing to obtain the pharmaceutical preparation; preferably, when dissolving each component in the solvent, dissolving the pH regulator in the solvent at the same time or prior to the ligand-drug conjugate or the pharmaceutically acceptable salt thereof.

In another preferred embodiment, a preparation method for preparing the above-mentioned pharmaceutical preparation comprises the following steps: uisng a prescribed amount of a ligand-drug conjugate or a pharmaceutically acceptable salt thereof, a freeze-dried excipient, a pH regulator, and a solvent, dissolving each component in the solvent in batches, regulating the pH value to 5.0 to 8.0 using an acidic substance or an alkaline substance in the pH regulator, and carrying out uniform mixing to obtain the pharmaceutical preparation; preferably, when dissolving each component in the solvent, dissolving the pH regulator in the solvent at the same time or prior to the ligand-drug conjugate or the pharmaceutically acceptable salt thereof.

In one embodiment, the preparation method for preparing the above-mentioned pharmaceutical preparation is not carried out in a dark place.

In one embodiment, the preparation method for preparing the above-mentioned pharmaceutical preparation is carried out in a dark place.

In one embodiment, the preparation method for preparing the above-mentioned pharmaceutical preparation is carried out at a temperature of 15°C to 40°C, such as 15°C, 20°C, 25°C, 30°C, 35°C, or 40°C.

In one embodiment, the preparation method for preparing the above-mentioned pharmaceutical preparation is carried out under nitrogen protection.

In one embodiment, the preparation method for preparing the above-mentioned pharmaceutical preparation is not carried out under nitrogen protection.

In one preferred embodiment, the preparation method for preparing the above-mentioned pharmaceutical preparation is carried out in a dark place, at a temperature of 15°C to 40°C, and under no nitrogen protection.

### [Freeze-dried preparation and preparation method therefor]

The present invention provides a freeze-dried preparation prepared by freeze-drying the pharmaceutical preparation of the present invention.

The present invention further provides a method for preparing the above-mentioned freeze-dried preparation, comprising the following steps: freeze-drying the pharmaceutical preparation of the present invention to obtain the freeze-dried preparation.

In one embodiment, in the above-mentioned preparation method, the freeze-drying comprises the following steps: freezing the pharmaceutical preparation at - 45°C; carrying out first drying on the pharmaceutical preparation at -15°C to -5°C; carrying out second drying on the pharmaceutical preparation at 15°C to 25°C.

In one preferred embodiment, in the above-mentioned preparation method, the freeze-drying comprises the following steps: freezing the pharmaceutical preparation at -45°C; carrying out first drying on the pharmaceutical preparation at -5°C; carrying out second drying on the pharmaceutical preparation at 15°C.

In another preferred embodiment, in the above-mentioned preparation method, the freeze-drying comprises the following steps: freezing the pharmaceutical preparation at -45°C; carrying out first drying on the pharmaceutical preparation at - 10°C; carrying out second drying on the pharmaceutical preparation at 15°C.

In still another preferred embodiment, in the above-mentioned preparation method, the freeze-drying comprises the following steps: freezing the pharmaceutical preparation at -45°C; carrying out first drying on the pharmaceutical preparation at - 15°C; carrying out second drying on the pharmaceutical preparation at 25°C.

In one more preferred embodiment, in the above-mentioned preparation method, the freeze-drying comprises the following steps: freezing the pharmaceutical preparation at -45°C for 120 min; carrying out first drying on the pharmaceutical preparation at -15°C for 720 min; carrying out second drying on the pharmaceutical preparation at 25°C for 720 min.

In one embodiment, the above-mentioned freeze-dried preparation is stable at room temperature.

### [Liquid preparation and preparation method therefor]

The present invention provides a liquid preparation reconstituted by the freeze-dried preparation of the present invention using water.

The present invention further provides a preparation method for preparing the above-mentioned liquid preparation, comprising the following steps: mixing the freeze-dried preparation of the present invention with water to obtain the liquid preparation.

In one embodiment, in the above-mentioned preparation method, the reconstitution is carried out using distilled water, pure water, or sterile water.

In one embodiment, the freeze-dried preparation has a pH value of 5.0 to 8.0 after being reconstituted using water.

### [Device]

The present invention provides a drug-containing delivery device, comprising one of the above-mentioned pharmaceutical preparation, the above-mentioned freeze-dried preparation, or the above-mentioned liquid preparation.

The present invention further provides a pre-filled syringe, comprising one of the above-mentioned pharmaceutical preparation, the above-mentioned freeze-dried preparation, or the above-mentioned liquid preparation.

### [Medical use]

The present invention provides the use of the above-mentioned pharmaceutical preparation comprising an active drug, the above-mentioned freeze-dried preparation, or the above-mentioned liquid preparation in the preparation of a delivery device or a pre-filled syringe or a drug.

In one embodiment, the delivery device or pre-filled syringe or drug in the above-mentioned use is used for enhancing an immune effector cell response in a subject.

In one embodiment, the delivery device or pre-filled syringe or drug in the above-mentioned use is used for reducing immunosuppression in a subject.

In one embodiment, the delivery device or pre-filled syringe or drug in the above-mentioned use is used for the treatment and/or prevention of a cancer in a subject.

Further, the cancer in the above-mentioned use comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, testicular cancer, skin cancer, lymphoma, and multiple myeloma.

In one embodiment, the delivery device or pre-filled syringe or drug in the above-mentioned use is used for the treatment and/or prevention of an immune disease in a subject.

Further, the immune disease in the above-mentioned use comprises one or more of a connective tissue disease, systemic sclerosis, rheumatoid arthritis, and systemic lupus erythematosus.

In one embodiment, the delivery device or pre-filled syringe or drug in the above-mentioned use is used for the treatment and/or prevention of a cardiovascular disease in a subject.

Further, the cardiovascular disease in the above-mentioned use comprises one or more of angina pectoris, myocardial infarction, apoplexy, heart attack, a hypertensive heart disease, a rheumatic heart disease, cardiomyopathy, cardiac arrhythmia, and a congenital heart disease.

In one embodiment, the delivery device or pre-filled syringe or drug in the above-mentioned use is used for the treatment and/or prevention of a metabolic disease in a subject.

Further, the metabolic disease in the above-mentioned use comprises one or more of diabetes mellitus, gout, obesity, hypoglycemia, hyperglycemia, and dyslipidemia.

In one embodiment, the delivery device or pre-filled syringe or drug in the above-mentioned use is used for the treatment and/or prevention of a neurological disease in a subject.

Further, the neurological disease in the above-mentioned use comprises one or more of Alzheimer's disease, Parkinson's disease, Huntington's disease, head injury, multiple sclerosis, vertigo, coma, and epilepsy.

The technical solutions of the present invention are further described below in conjunction with particular examples.

### Example 1

### 1.1 Screening of type of pH regulator

CR19213 is poorly soluble in water and was unstable to acids and alkalis, and therefore a suitable pH regulator is required to be added in a drug prescription containing CR19213 so as to ensure the preparation and stability of a product.

Injection-grade pH regulators are mainly buffer salts. Common Tris, acetate, citrate and phosphate were selected, with a tentative concentration of 0.5%; since CR19213 was sensitive to both acids and alkalis, the pH value of the buffer salt solution was tentatively 7.0 (regulated with 1 M hydrochloric acid or sodium hydroxide); and the freeze-dried excipient was tentatively common 5% mannitol. Samples were prepared by dissolving CR19213 in the above-mentioned buffer salt solutions, respectively, and subjected to 10-day influencing factor testing of high temperature (40°C) and strong light (4500 lx, near ultraviolet light of 85 µw/cm²) to investigate the characters, acidity, re-dissolution time, moisture, character after re-dissolution, content, and related substances of the product.

### 1.1.1 Prescription

**Table 1 Composition of prescription for screening of type of pH regulator**

| **Name** | **Batch No.** | | | |
|---|---|---|---|---|
| | **Prescription 1** | **Prescription 2** | **Prescription 3** | **Prescription 4** |
| CR19213 | 10.0 mg | 10.0 mg | 10.0 mg | 10.0 mg |
| Mannitol | 100 mg | 100 mg | 100 mg | 100 mg |
| 0.5% Tris solution (pH 7.0) | To 2 mL | / | / | / |
| 0.5% sodium acetate solution (pH 7.0) | / | To 2 mL | / | / |
| 0.5% sodium citrate solution (pH 7.0) | / | / | To 2 mL | / |
| 0.5% sodium dihydrogen phosphate solution (pH 7.0) | / | / | / | To 2 mL |

### 1.1.2 Process

(1) a 0.5% Tris solution, a 0.5% sodium acetate solution, a 0.5% sodium citrate solution, and a 0.5% sodium dihydrogen phosphate solution with pH 7.0 (regulated with 0.1 M hydrochloric acid or sodium hydroxide) were respectively prepared for later use;
(2) a prescribed amount of mannitol was respectively weighed, 70% of the total amount of the corresponding buffer salt solution was added, stirring was carried out for dissolution, then a prescribed amount of raw material CR19213 was added, and stirring was carried out for dissolution in a dark place at room temperature;
(3) the corresponding amount of the buffer salt solution was supplemented to the total amount, respectively;
(4) filling was carried out at 2 mL/vial, and half of a stopper was pressed into the vial;
(5) freeze-drying was carried out, and the stopper was pressed under vacuum condition; and
(6) capping was carried to obtain the product.

**Table 2 Freeze-drying parameter settings**

| **Stage** | **Shelve temperature (°C)** | **Heating time (min)** | **Holding time (min)** | **Pressure control (mbar)** |
|---|---|---|---|---|
| Pre-freezing | -45 | 50 | 120 | / |
| Pre-vacuumizing | / | / | / | 0.2 |
| First drying | -15 | 150 | 780 | 0.2 |
| Second drying | 25 | 200 | 720 | / |

### 1.1.3 Detection results

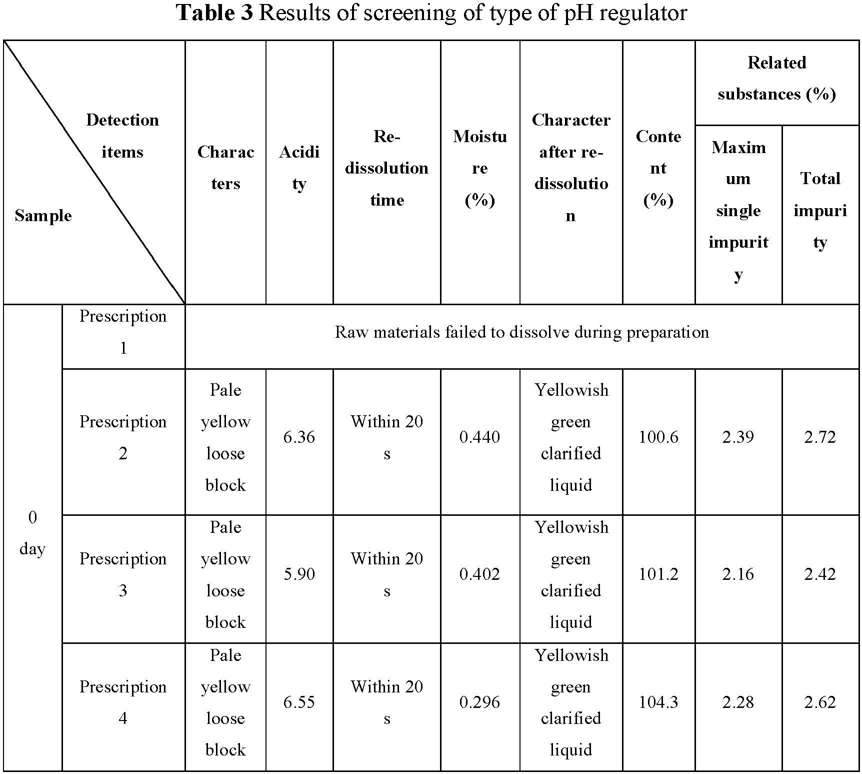

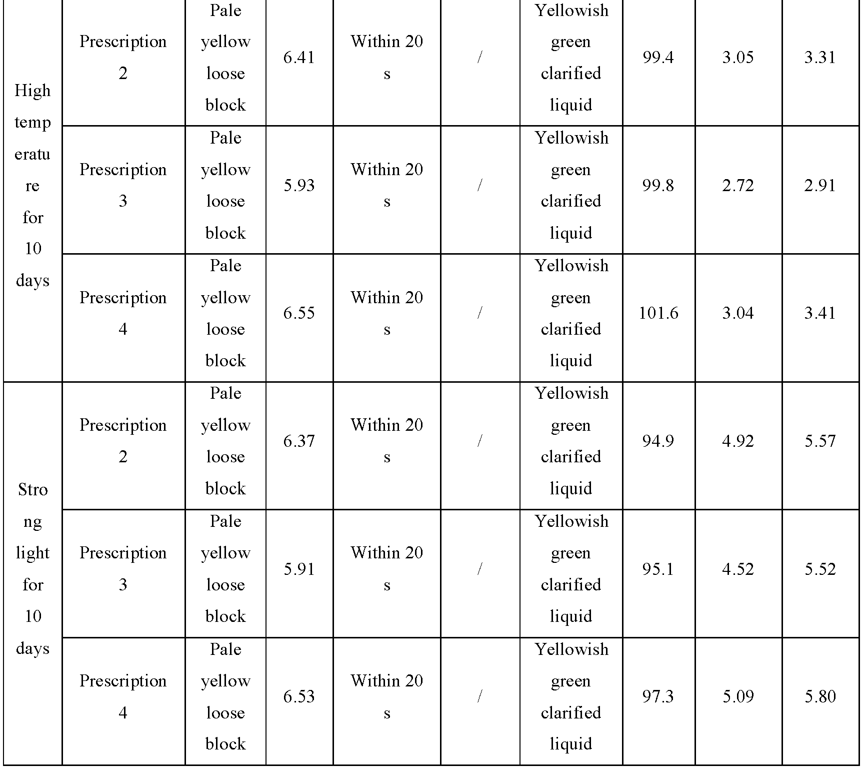

The results showed that: CR19213 was insoluble in prescription 1 (pH 7.0, 0.5% Tris solution), but was soluble in the remaining prescriptions. The investigation results of the 10-day influencing factor testing of high temperature (40°C) and strong light (4500 lx, near ultraviolet light of 85 µw/cm²) showed that the finished product was degraded to some extent under the high temperature condition and was significantly degraded under the strong light condition (the degradation under high temperature and strong light was mainly related to the sensitivity of CR19213 to temperature and strong light). Prescription 3 (pH 7.0, 0.5% sodium citrate solution) had the lowest level of impurities, and therefore the pH regulator was preferably sodium citrate.

### 1.2 Screening of pH value

pH value is of particular importance for the stability of a drug, and therefore during the preparation of an injection intermediate, the pH value must be controlled as necessary to ensure stable quality of the injection during freeze-drying and storage.

An experiment was designed to regulate the pH values of intermediate solutions to 4.0, 5.0, 5.5, 6.0, 6.5, 7.0, and 8.0, respectively, and the pH value, content, and related substances of the finished product were used as main investigation indicators to compare the quality of the finished product.

### 1.2.1 Prescription

**Table 4 Composition of prescription for screening of pH value**

| **Name** | **Prescrip tion 13** | **Prescrip tion 14** | **Prescrip tion 15** | **Prescrip tion 16** | **Prescrip tion 17** | **Prescrip tion 18** | **Prescrip tion 19** |
|---|---|---|---|---|---|---|---|
| CR19213 | 10.0 mg | 10.0 mg | 10.0 mg | 10.0 mg | 10.0 mg | 10.0 mg | 10.0 mg |
| Sodium citrate | 11.4 mg | 11.4 mg | 11.4 mg | 11.4 mg | 11.4 mg | 11.4 mg | 11.4 mg |
| Mannitol | 100 mg | 100 mg | 100 mg | 100 mg | 100 mg | 100 mg | 100 mg |
| Hydrochloric acid/sodium hydroxide | Q.S. | Q.S. | Q.S. | Q.S. | Q.S. | Q.S. | Q.S. |
| Water | 2 mL | 2 mL | 2 mL | 2 mL | 2 mL | 2 mL | 2 mL |
| pH value | 4.0 | 5.0 | 5.5 | 6.0 | 6.5 | 7.0 | 8.0 |

### 1.2.2 Process

(1) 70% of a prescribed amount of water for injection was added into a preparation container, a prescribed amount of sodium citrate was added, and stirring was carried out for dissolution;
(2) a prescribed amount of mannitol was weighed and added into the preparation container; and stirring was carried out for dissolution, then a prescribed amount of CR19213 was added, and stirring was carried out for dissolution in a dark place at room temperature;
(3) the pH was regulated to a selected value with hydrochloric acid/sodium hydroxide and water was added to the total amount;
(4) filling was carried out at 2 mL/vial, and half of a stopper was pressed into the vial;
(5) freeze-drying was carried out, and the stopper was pressed under vacuum condition; and the specific parameters of the freeze-drying were as shown in Table 2; and
(6) capping was carried to obtain the product.

### 1.2.3 Investigation results

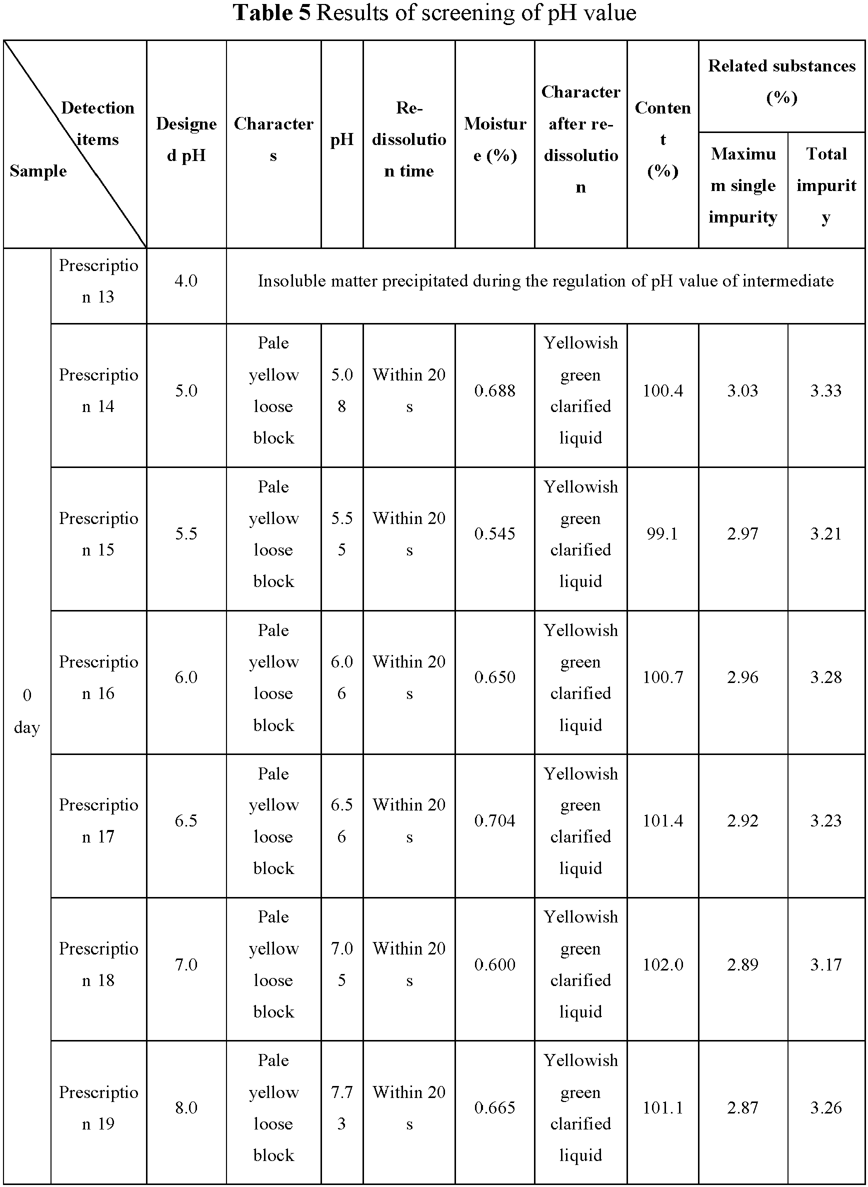

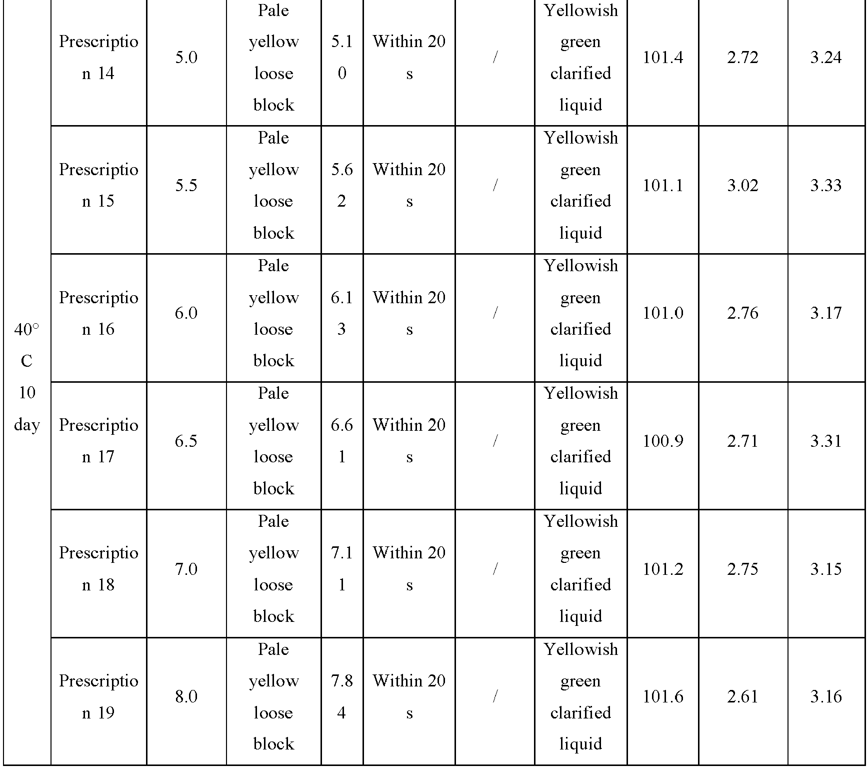

The results showed that: insoluble matter precipitated in prescription 13 (pH 4.0) during the preparation; and the samples in the remaining prescriptions (intermediate with pH 5.0 to 8.0) were of comparable quality. The investigation results of the 10-day influencing factor testing of 40°C showed that there was no significant change in the product quality. Since the pH value was about 6.0 (without acid or alkali regulation) after raw materials and adjuvants were added, the quality of the product at this acidity was stable. Therefore, it was determined that the target acidity value of the intermediate was pH 6.0 and the acceptable range was 5.5 to 6.5.

### 1.3 Screening of amount of pH regulator

The solubility of CR19213 is related to the pH value of a solution, and the amount of the pH regulator (expressed as the concentration of a buffer salt) directly influences the pH value of the solution. Therefore, the concentration of the buffer salt needs to be screened. Since CR19213 was added to the sodium citrate solution with pH 7.0, and completely dissolved to form an acidic solution, a 1.0% sodium citrate solution (11.4 mg/mL) with pH 7.5 (regulated with citric acid) was prepared in order to make the finished product be more near-neutral, and diluted with water to obtain sodium citrate solutions at concentrations of 0.1%, 0.25%, and 0.5%, respectively. Intermediate solutions were prepared using the above-mentioned sodium citrate solutions with different concentrations. The dissolution in the intermediate solutions during the preparation were investigated.

### 1.3.1 Prescription

**Table 6 Composition of prescription for screening of amount of pH regulator**

| **Name** | **Batch No.** | | | |
|---|---|---|---|---|
| | **Prescription 5** | **Prescription 6** | **Prescription 7** | **Prescription 8** |
| CR19213 | 10.0 mg | 10.0 mg | 10.0 mg | 10.0 mg |
| Mannitol | 100 mg | 100 mg | 100 mg | 100 mg |
| 0.1% sodium citrate solution | To 2 mL | / | / | / |
| 0.25% sodium citrate solution | / | To 2 mL | / | / |
| 0.5% sodium citrate solution | / | / | To 2 mL | / |
| 1.0% sodium citrate solution | / | / | / | To 2 mL |

### 1.3.2 Process

(1) a 1.0% sodium citrate solution with pH 7.5 (regulated with citric acid) was prepared for later use;
(2) dilution was carried out with water to obtain 0.1%, 0.25% and 0.5% sodium citrate solutions, respectively;
(3) a prescribed amount of mannitol was weighed, 70% of the total amount of the sodium citrate solution was added, stirring was carried out for dissolution, then a prescribed amount of CR19213 was added, and stirring was carried out for dissolution in a dark place at room temperature; and
(4) the corresponding amount of the sodium citrate solution was supplemented to the total amount to obtain the product.

### 1.3.3 Investigation results

**Table 7 Results of investigation of amount of pH regulator**

| **Batch No.** | **Concentration of sodium citrate solution** | **Dissolution phenomenon** | **Solution character** |
|---|---|---|---|
| Prescription 5 | 0.1% | Much insoluble matter | Suspension |
| Prescription 6 | 0.25% | Dissolved after stirred for 3 hours | Yellowish green clear liquid |
| Prescription 7 | 0.5% | Dissolved after stirred for 20 minutes | Yellowish green clear liquid |
| Prescription 8 | 1.0% | Dissolved after stirred for 12 minutes | Yellowish green clear liquid |

The dissolution phenomenon showed that the sodium citrate solution with the concentration of 0.1% could not completely dissolve CR19213, the sodium citrate solution with the concentration of 0.25% (prescription 6) dissolved CR19213 after 3 hours, and the sodium citrate solution with the concentration of 0.5% (prescription 7) and the sodium citrate solution with the concentration of 1.0% (prescription 8) could dissolve CR19213 within half an hour.

The detection results showed that the 0.5% sodium citrate solution had a pH value of 8.42, and after mannitol was added and CR19213 was dissolved, it had a pH value of 5.95. Since the bulk drug was acidic, the pH value may be not pre-regulated with citric acid in order to make the finished product be more near-neutral.

### Example 2

In order to obtain a freeze-dried product which is aesthetic, easy to redissolve and good in stability, a protectant needs to be screened. Common mannitol, sucrose, lactose and sorbitol were selected as freeze-dried excipients for comparative investigation.

### 2.1 Prescription

**Table 8 Composition of prescription for screening of freeze-dried excipient**

| **Name** | **Prescription 9** | **Prescription 10** | **Prescription 11** | **Prescription 12** |
|---|---|---|---|---|
| CR19213 | 10.0 mg | 10.0 mg | 10.0 mg | 10.0 mg |
| Sodium citrate (dihydrate) | 11.4 mg | 11.4 mg | 11.4 mg | 11.4 mg |
| Mannitol | 100 mg | 80 mg | / | / |
| Sucrose | / | 20 mg | / | / |
| Lactose | / | / | 100 mg | / |
| Sorbitol | / | / | / | 100 mg |
| Hydrochloric acid/sodium hydroxide | Q.S. | Q.S. | Q.S. | Q.S. |
| Water for injection to | 2 mL | 2 mL | 2 mL | 2 mL |

### 2.2 Process

(1) 70% of a prescribed amount of water for injection was added into a preparation container, a prescribed amount of sodium citrate (dihydrate) was added, and stirring was carried out for dissolution;
(2) a prescribed amount of a freeze-dried excipient was weighed and added into the preparation container; and stirring was carried out for dissolution, then a prescribed amount of CR19213 was added, and stirring was carried out for dissolution in a dark place at room temperature;
(3) if necessary, the pH was regulated to 5 to 7 with 0.1 M hydrochloric acid/sodium hydroxide and water was added to the total amount;
(4) filling was carried out at 2 mL/vial, and half of a stopper was pressed into the vial;
(5) freeze-drying was carried out, and the stopper was pressed under vacuum condition; and the specific parameters of the freeze-drying were as shown in Table 2; and
(6) capping was carried to obtain the product.

### 2.3 Investigation results

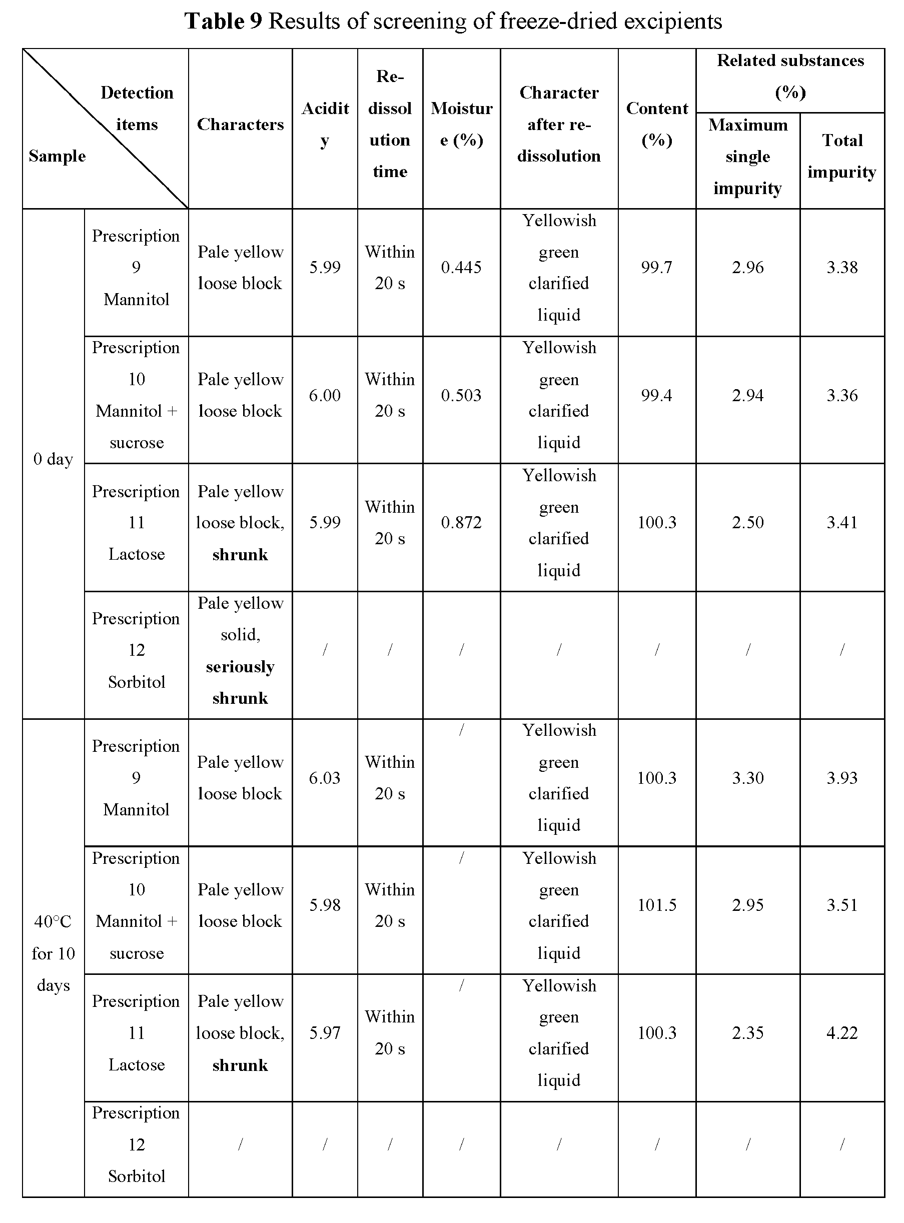

The results showed that: when lactose (prescription 11) and sorbitol (prescription 12) were used as the excipients for the product, the finished product was shrunk. The investigation results of the 10-day influencing factor testing of 40°C showed that the finished products in prescription 9 and prescription 10 were of comparable product quality.

### Example 3

The prescription determined by the study was selected for process investigation to obtain a freeze-dried preparation for injection which can be stably prepared.

**Table 10 Composition of prescription for investigating production process**

| **Name** | **Amount** |
|---|---|
| CR19213 | 10.0 mg |
| Sodium citrate | 11.4 mg |
| Mannitol | 100 mg |
| Hydrochloric acid/sodium hydroxide | Q.S. |
| Water for injection to | 2 mL |

### 3.1 Investigation of dosing sequence

Different adjuvants have different acidity-alkalinity and different stability. The adding sequence of raw materials and adjuvants may have an effect on the quality of an injection. Different dosing sequences of raw materials and adjuvants were designed, and the influence of the dosing sequences of the raw materials and various adjuvants on the product quality was investigated.

**Table 11 Investigation of different dosing sequences of CR19213 for injection**

| **Process No.** | | **Dosing process** |
|---|---|---|
| Small-scale experiment | Process 1 | Mannitol and sodium citrate were firstly added, stirring was carried out for dissolution, then a prescribed amount of CR19213 was added, and stirring was carried out for dissolution in a dark place at room temperature. |
| | Process 2 | Sodium citrate was firstly added and dissolved, then CR19213 was added, finally mannitol was added, and stirring was carried out for dissolution in a dark place at room temperature. |
| | Process 3 | Mannitol was firstly added and dissolved, then CR19213 was added, finally sodium citrate was added, and stirring was carried out for dissolution in a dark place at room temperature. |
| | Process 4 | Mannitol, sodium citrate, and CR19213 were added at the same time, and stirring was carried out for dissolution in a dark place at room temperature. |

The quality attributes of the intermediate output by the dosing process were the content, acidity, characters, and related substances.

**Table 12 Results of investigation of different adding sequence of raw materials and adjuvants**

| **Sample** | **Process 1** | **Process 2** | **Process 3** | **Process 4** |
|---|---|---|---|---|
| Characters of intermediate | Yellowish green clarified liquid | Yellowish green clarified liquid | Yellowish green clarified liquid; CR19213 was insoluble and dissolved 1 hour after adding sodium citrate | Yellowish green clarified liquid |
| Acidity | 6.02 | 6.03 | 6.02 | 5.96 |
| Content (%) | 99.9 | 99.0 | 99.7 | 100.2 |

The results showed that different dosing sequences resulted in comparable characters, acidity and content of the intermediate solution.

### 3.2 Dispensing environment

The dispensing environment comprises the oxygen content of the solution, the light environment to which the dispensing is exposed, and the dispensing temperature and time.

### 3.2.1 Oxygen content of solution

CR19213 is sensitive to 1% H₂O₂ and easily oxidized in solution. Therefore, whether the product needs nitrogen protection during the preparation or not needs to be investigated to reduce the oxygen content.

The intermediate solution was prepared with and without a nitrogen protection process respectively, and placed for 24 hours to investigate the stability of the intermediate solution. Whether there were significant differences in the characters, content, pH and related substances of the intermediate solution was mainly investigated.

### 3.2.1.1 Process

**Table 13 Process for investigating oxygen content of solution**

| **Process** | **Process procedure** |
|---|---|
| Process 5 Without nitrogen filling | (1) 70% of a prescribed amount of water for injection was added, a prescribed amount of an adjuvant was added, and stirring was carried out for dissolution; |
| | (2) a prescribed amount of CR19213 was added and stirring was carried out for dissolution in a dark place at room temperature; |
| | (3) if necessary, the pH was regulated to 5.5 to 6.5 with 0.1 M hydrochloric acid/sodium hydroxide and water was added to the total amount; and |
| | (4) the intermediate solution was placed in a dark place at room temperature for 24 hours, and the intermediate solution was sampled and detected at 0 hour, 8 hours, 20 hours, and 24 hours. |
| Process 6 With nitrogen filling | (1) 70% of a prescribed amount of water for injection was added, nitrogen filling was continuously carried out for 30 minutes, a prescribed amount of an adjuvant was added, and stirring was carried out for dissolution; |
| | (2) a prescribed amount of CR19213 was added and stirring was carried out for dissolution in a dark place at room temperature under nitrogen filling conditions; |
| | (3) if necessary, the pH was regulated to 5.5 to 6.5 with 0.1 M hydrochloric acid/sodium hydroxide and water was added to the total amount; and |
| | (4) the intermediate solution was placed in a dark place at room temperature under nitrogen filling conditions for 24 hours, and the intermediate solution was sampled and detected at 0 hour, 8 hours, 20 hours, and 24 hours. |

### 3.2.1.2 Investigation results

**Table 14 Results of investigation of nitrogen protection process**

| **Sample** | | | **Process 5 Without nitrogen filling** | **Process 6 With nitrogen filling** |
|---|---|---|---|---|
| Characters | | 0 hour | Yellowish green clarified liquid | Yellowish green clarified liquid |
| | | 8 hour | Yellowish green clarified liquid | Yellowish green clarified liquid |
| | | 20 hour | Yellowish green clarified liquid | Yellowish green clarified liquid |
| | | 24 hour | Yellowish green clarified liquid | Yellowish green clarified liquid |
| pH | | 0 hour | 6.07 | 6.06 |
| | | 8 hour | 6.07 | 6.06 |
| | | 20 hour | 6.08 | 6.07 |
| | | 24 hour | 6.09 | 6.08 |
| Content (%) | | 0 hour | 100.5 | 101.1 |
| | | 8 hour | 101.0 | 101.7 |
| | | 20 hour | 100.4 | 100.2 |
| | | 24 hour | 100.8 | 100.8 |
| Related substances (%) | Maximum single impurity | 0 hour | 2.15 | 2.09 |
| | | 8 hour | 2.08 | 2.19 |
| | | 20 hour | 2.02 | 2.07 |
| | | 24 hour | 2.01 | 2.05 |
| | Total impurity | 0 hour | 2.76 | 2.67 |
| | | 8 hour | 2.58 | 2.74 |
| | | 20 hour | 2.76 | 2.73 |
| | | 24 hour | 2.50 | 2.52 |

The result showed that there was no significant difference in the characters, content, pH and related substances between the drug liquid intermediate with nitrogen filling and the drug liquid intermediate without nitrogen filling within 24 hours, indicating that the nitrogen protection process was not needed.

### 3.2.2 Investigation of influence of illumination on dispensing

Raw material CR19213 is sensitive to illumination. In view of that it would come into with indoor light during actual dispensing, in order to investigate the influence of illumination during preparation, an experiment was designed to investigate the influence of indoor light on the product stability during the dispensing.

The intermediate solution was prepared and placed in a dark place and under indoor light illumination conditions for 24 hours, respectively. The characters, pH, content and related substances of the drug liquid intermediate were detected.

**Table 15 Results of investigation of influence of illumination during preparation**

| **Process** | | | **Process 7** | **Process 8** |
|---|---|---|---|---|
| **Illumination condition** | | | **In a dark place** | **Not in a dark place** |
| Characters | | 0 hour | Yellowish green clarified liquid | |
| | | 8 hour | Yellowish green clarified liquid | Yellowish green clarified liquid |
| | | 20 hour | Yellowish green clarified liquid | Yellowish green clarified liquid |
| | | 24 hour | Yellowish green clarified liquid | Yellowish green clarified liquid |
| pH | | 0 hour | 6.07 | |
| | | 8 hour | 6.07 | 6.06 |
| | | 20 hour | 6.08 | 6.07 |
| | | 24 hour | 6.09 | 6.08 |
| Content (%) | | 0 hour | 100.5 | |
| | | 8 hour | 101.0 | 101.0 |
| | | 20 hour | 100.4 | 100.8 |
| | | 24 hour | 100.8 | 101.0 |
| Related substances (%) | Maximum single impurity | 0 hour | 2.15 | |
| | | 8 hour | 2.08 | 2.05 |
| | | 20 hour | 2.02 | 2.06 |
| | | 24 hour | 2.01 | 2.13 |
| | Total impurity | 0 hour | 2.76 | |
| | | 8 hour | 2.58 | 2.55 |
| | | 20 hour | 2.76 | 2.65 |
| | | 24 hour | 2.50 | 2.69 |

The results showed that: there was no significant difference in the characters, pH, content and related substances within 24 hours between the intermediate solution under the indoor light condition and the intermediate solution in a dark place.

### 3.2.3 Dispensing temperature control

CR19213 is sensitive to temperature. To determine the temperature control during the production and preparation, the prepared intermediate solution was kept at 15°C, 25°C and 40°C, respectively, and sampled and detected at 0 hour, 8 hours, 20 hours and 24 hours. The influence of the temperature on the characters, pH, content and related substances of the intermediate solution was investigated.

**Table 16 Investigation of stability of intermediate solution at different temperatures**

| **Process** | | | **Process 9** | **Process 10** | **Process 11** |
|---|---|---|---|---|---|
| **Temperature at the time of placement** | | | **15°C** | **25°C** | **40°C** |
| Characters | | 0 hour | Yellowish green clarified liquid | | |
| | | 8 hour | Yellowish green clarified liquid | Yellowish green clarified liquid | Yellowish green clarified liquid |
| | | 20 hour | Yellowish green clarified liquid | Yellowish green clarified liquid | Yellowish green clarified liquid |
| | | 24 hour | Yellowish green clarified liquid | Yellowish green clarified liquid | Yellowish green clarified liquid |
| pH | | 0 hour | 6.12 | | |
| | | 8 hour | 6.11 | 6.11 | 6.10 |
| | | 20 hour | 6.10 | 6.11 | 6.10 |
| | | 24 hour | 6.09 | 6.11 | 6.10 |
| Content (%) | | 0 hour | 102.5 | | |
| | | 8 hour | 102.5 | 102.8 | 102.8 |
| | | 20 hour | 102.5 | 103.4 | 102.9 |
| | | 24 hour | 102.3 | 102.9 | 102.4 |
| Related substan ces (%) | Maximu m single impurity | 0 hour | 1.92 | | |
| | | 8 hour | 2.09 | 2.04 | 1.98 |
| | | 20 hour | 1.99 | 1.96 | 1.92 |
| | | 24 hour | 1.92 | 1.95 | 1.92 |
| | Total impurity | 0 hour | 2.50 | | |
| | | 8 hour | 2.50 | 2.32 | 2.53 |
| | | 20 hour | 2.44 | 2.41 | 2.25 |
| | | 24 hour | 2.47 | 2.65 | 2.20 |

The results showed that there was no significant change in the characters, pH, content and related substances of the intermediate solution after the intermediate solution was kept at 15°C, 25°C and 40°C for 24 hours.

### 3.3 Freeze-drying parameters

To better optimize the freeze-drying parameters, the prescription solution was subjected to DSC detection. The results were as shown in FIG. 1.

The DSC detection results showed a eutectic point of -19.72°C and a co-melting point of -3.90°C. Therefore, it was determined that the pre-freezing temperature was - 45°C (about 25 °C below the eutectic point).

The freeze-drying process parameters, mainly the first drying temperature and the second drying temperature, were investigated. The first drying temperatures were selected to be -15°C, -10°C and -5°C, respectively, and the second drying temperatures were selected to be 25°C, 15°C and 15°C, respectively. Samples were prepared, and the characters, acidity, moisture, re-dissolution time, character after re-dissolution, content and related substances of the product were investigated.

**Table 17 Freeze-drying parameter settings**

| **Process** | | **Process 12** | **Process 13** | **Process 14** |
|---|---|---|---|---|
| Pre-freezing | Shelve temperature (°C) | -45 | -45 | -45 |
| | Heating time (min) | 50 | 50 | 50 |
| | Holding time (min) | 120 | 120 | 120 |
| | Pressure control (mbar) | / | / | / |
| First drying | Shelve temperature (°C) | **-15** | **-10** | **-5** |
| | Heating time (min) | 150 | 150 | 150 |
| | Holding time (min) | 720 | 720 | 720 |
| | Pressure control (mbar) | 0.2 | 0.2 | 0.2 |
| Second drying | Shelve temperature (°C) | **25** | **15** | **15** |
| | Heating time (min) | 200 | 200 | 200 |
| | Holding time (min) | 720 | 720 | 720 |
| | Pressure control (mbar) | / | / | / |

**Table 18 Results of investigation of optimization of freeze-drying parameters**

| **Freeze-drying process** | | **Process 12** | **Process 13** | **Process 14** |
|---|---|---|---|---|
| Characters | | Pale yellow loose block | Pale yellow loose block | Pale yellow loose block |
| Acidity | | 6.08 | 6.07 | 6.09 |
| Moisture (%) | | 0.754 | 0.898 | 1.464 |
| Re-dissolution time | | Within 20 s | Within 20 s | Within 20 s |
| Character after re-dissolution | | Yellowish green clarified liquid | Yellowish green clarified liquid | Yellowish green clarified liquid |
| Content (%) | | 100.6 | 100.9 | 101.3 |
| Related substances | %Maximum single impurity | 2.05 | 2.07 | 2.28 |
| | %Total impurity | 2.41 | 2.51 | 2.83 |

The results showed that: there was no significant difference in the characters, acidity, moisture, re-dissolution time, character after re-dissolution, content and related substances among the samples prepared by freeze-drying processes 12, 13 and 14. The sample prepared by process 12 had relatively less moisture, and therefore process 12 was selected as the freeze-drying parameters of CR19213 for injection.

**Table 19 Determined freeze-drying parameters**

| **Stage** | **Shelve temperature (°C)** | **Heating time (min)** | **Holding time (min)** | **Pressure control (mbar)** |
|---|---|---|---|---|
| Pre-freezing | -45 | 50 | 120 | / |
| First drying | -15 | 150 | 720 | 0.2 |
| Second drying | 25 | 200 | 720 | / |

### Example 4

The composition of the prescription determined by the study was selected for stability investigation.

**Table 20 Composition of prescription**

| **Name** | **Amount** |
|---|---|
| CR19213 | 10.0 mg |
| Sodium citrate | 11.4 mg |
| Mannitol | 100 mg |
| Water for injection to | 2 mL |

A CR19213 freeze-dried preparation was prepared according to the above-mentioned prescription and the processes in examples 1-3, and placed at 25°C. The stability detection results were as follows:

**Table 21 Stability of freeze-dried pharmaceutical preparation of determined prescription and process**

| **Test items** | | **Stability at 25°C** | | | | |
|---|---|---|---|---|---|---|
| | | **0 month** | **1 month** | **2 months** | **3 months** | **6 months** |
| Characters | | Pale yellow loose block | Pale yellow loose block | Pale yellow loose block | Pale yellow loose block | Pale yellow loose block |
| Moisture | | 0.8% | 2.1% | 2.1% | 2.2% | 2.2% |
| pH value | | N/A | 6.1 | 5.6 | 6.1 | 5.9 |
| Related substances | | 4.1% | 4.4% | 5.2% | 4.3% | 4.9% |
| Content determination | | 103.1% | 105.0% | 105.0% | 105.0% | 1026% |
| Insoluble particle | ≥ 10 µm | 0 | 12 particles/vial | 30 particles/vial | 12 particles/vial | 8 particles/vial |
| | ≥ 25 µm | 0 | 0 particles/vial | 0 particles/vial | 0 particles/vial | 2 particles/vial |

The results showed that there was no obvious change in the characters, moisture, pH value, related substances, content, and insoluble particles within 6 months at 25°C, indicating that the determined prescription and process can obtain the product that can be stably stored at 25°C for at least 6 months.

## Claims

1. A pharmaceutical preparation, **characterized in that** the pharmaceutical preparation comprises an active drug and optionally a pharmaceutically acceptable adjuvant.

2. The pharmaceutical preparation according to claim 1, **characterized in that**
the active drug comprises a ligand-drug conjugate or a pharmaceutically acceptable salt thereof;
the pharmaceutically acceptable adjuvant comprises one or more of a freeze-dried excipient and a pH regulator.

3. The pharmaceutical preparation according to claim 1 or 2, **characterized in that**
the active drug comprises a ligand-drug conjugate or a pharmaceutically acceptable salt thereof;
the pharmaceutically acceptable adjuvant comprises one or more of a freeze-dried excipient, a pH regulator, and a solvent.

4. The pharmaceutical preparation according to claim 3, **characterized in that** the solvent is water;
preferably, the solvent is purified water;
more preferably, the purified water is sterile water, distilled water, or deionized water;
and more preferably, the sterile water is sterile water for injection, single-distilled water, or double-distilled water.

5. The pharmaceutical preparation according to claim 3, **characterized in that**
the active drug has a concentration of 2 mg/mL to 8 mg/mL, such as 2.0 mg/mL, 2.2 mg/mL, 2.4 mg/mL, 2.6 mg/mL, 2.8 mg/mL, 3.0 mg/mL, 3.2 mg/mL, 3.4 mg/mL, 3.6 mg/mL, 3.8 mg/mL, 4.0 mg/mL, 4.2 mg/mL, 4.4 mg/mL, 4.6 mg/mL, 4.8 mg/mL, 5.0 mg/mL, 5.2 mg/mL, 5.4 mg/mL, 5.6 mg/mL, 5.8 mg/mL, 6.0 mg/mL, 6.2 mg/mL, 6.4 mg/mL, 6.6 mg/mL, 6.8 mg/mL, 7.0 mg/mL, 7.2 mg/mL, 7.4 mg/mL, 7.6 mg/mL, 7.8 mg/mL, or 8.0 mg/mL.

6. The pharmaceutical preparation according to claim 2 or 3, **characterized in that**
the pH regulator comprises a buffer salt.

7. The pharmaceutical preparation according to claim 6, **characterized in that**
the pH regulator further comprises an acidic substance and/or an alkaline substance.

8. The pharmaceutical preparation according to claim 6, **characterized in that**
the buffer salt comprises one or more of an acetate, a phosphate, a citrate, or a hydrate thereof; preferably, each 1 molecule of the hydrate comprises 0.5, 1, 1.5, 2.0, 2.5 or 3 molecules of water;
and preferably, the buffer salt is one or more of sodium acetate, anhydrous sodium citrate, sodium citrate dihydrate or sodium dihydrogen phosphate.

9. The pharmaceutical preparation according to claim 7, **characterized in that**
the acidic substance comprises an acid; preferably, the acidic substance is one or more of hydrochloric acid, acetic acid, or citric acid;
the alkaline substance comprises an alkali or a salt; and preferably, the alkaline substance is one or more of sodium hydroxide, sodium carbonate or sodium bicarbonate.

10. The pharmaceutical preparation according to any one of claims 6 to 9, **characterized in that** when the buffer salt is selected from anhydrous sodium citrate or sodium citrate dihydrate, the buffer salt has a concentration of 2.85 mg/mL to 11.4 mg/mL, such as 2.85 mg/mL, 3.5 mg/mL, 4.2 mg/mL, 5.7 mg/mL, 6.5 mg/mL, 7.6 mg/mL, 8.5 mg/mL, 9.8 mg/mL, 10.5 mg/mL, or 11.4 mg/mL.

11. The pharmaceutical preparation according to claim 2 or 3, **characterized in that**
the freeze-dried excipient comprises a polyol, such as one or more of mannitol and xylitol;
preferably, the freeze-dried excipient is mannitol, preferably the mannitol has a concentration of 20 mg/mL to 150 mg/mL, such as 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, or 150 mg/mL; and more preferably the mannitol has a concentration of 40 mg/mL to 60 mg/mL.

12. The pharmaceutical preparation according to claim 11, **characterized in that**
the freeze-dried excipient further comprises a saccharide, such as one or more of a monosaccharide, a disaccharide, and a polysaccharide;
preferably, a mass ratio of the saccharide to the polyol is 1:1 to 1:5, such as 1:1, 1:1.5, 1:2, 1:2.5, 1:3, 1:3.5, 1:4, 1:4.5, 1:5;
and more preferably, a mass ratio of the saccharide to the polyol is 1:2 to 1:4.

13. The pharmaceutical preparation according to claim 12, **characterized in that**
the monosaccharide comprises one or more of glucose and fructose;
the disaccharide comprises one or more of sucrose, trehalose, and maltose;
and the polysaccharide comprises one or more of cyclodextrin and dextran.

14. The pharmaceutical preparation according to any one of claims 11 to 13, **characterized in that**
the freeze-dried excipient is mannitol and sucrose; and preferably, a mass ratio of the sucrose to the mannitol is 1:4.

15. The pharmaceutical preparation according to any one of claims 3 to 14, **characterized in that**
the pharmaceutical preparation has a pH value of 5.0 to 8.0; such as 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

16. The pharmaceutical preparation according to claim 1, **characterized in that** the pharmaceutical preparation comprises a ligand-drug conjugate or a pharmaceutically acceptable salt thereof, a freeze-dried excipient, a pH regulator, and a solvent; wherein the pharmaceutical preparation has a pH value of 5.0 to 8.0;
preferably, each 1 mL of the pharmaceutical preparation comprises 5 mg of a dual ligand-drug conjugate or a pharmaceutically acceptable salt thereof, 50 mg of mannitol, 5.7 mg of sodium citrate dihydrate, optionally an acidic substance or alkaline substance, and the balance made up of water; wherein the pharmaceutical preparation has a pH value of 5.0 to 8.0;
preferably, each 1 mL of the pharmaceutical preparation comprises 5 mg of a dual ligand-drug conjugate or a pharmaceutically acceptable salt thereof, 40 mg of mannitol, 10 mg of sucrose, 4.5 mg of anhydrous sodium citrate, optionally an acidic substance or alkaline substance, and the balance made up of water; wherein the pharmaceutical preparation has a pH value of 5.0 to 8.0;
preferably, each 1 mL of the pharmaceutical preparation comprises 4 mg of a dual ligand-drug conjugate or a pharmaceutically acceptable salt thereof, 50 mg of mannitol, an appropriate amount of sodium acetate, optionally an acidic substance or alkaline substance, and the balance made up of water; wherein the pharmaceutical preparation has a pH value of 5.0 to 8.0;
preferably, each 1 mL of the pharmaceutical preparation comprises 6 mg of a dual ligand-drug conjugate or a pharmaceutically acceptable salt thereof, 50 mg of mannitol, an appropriate amount of sodium dihydrogen phosphate, optionally an acidic substance or alkaline substance, and the balance made up of water; and wherein the pharmaceutical preparation has a pH value of 5.0 to 8.0.

17. The pharmaceutical preparation according to any one of claims 2 to 16, **characterized in that**
the ligand-drug conjugate is a dual ligand-drug conjugate;
preferably, the dual ligand-drug conjugate is a drug conjugate targeting a PSMA receptor and a TRPV6 receptor;
preferably, the dual ligand-drug conjugate has the following structure: 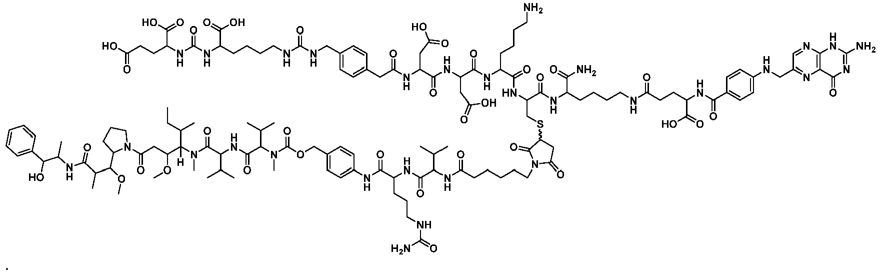
and more preferably, the dual ligand-drug conjugate has the following structure:

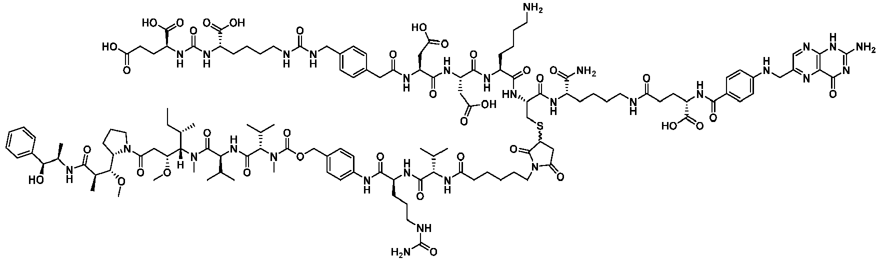

18. The pharmaceutical preparation according to any one of claims 1 to 17, **characterized in that** the pharmaceutical preparation is sterile.

19. The pharmaceutical preparation according to any one of claims 1 to 17, **characterized in that** the pharmaceutical preparation is stable during freezing and thawing.

20. A preparation method for preparing the pharmaceutical preparation according to any one of claims 1 to 19, **characterized in that** the preparation method comprises the following steps:
using a prescribed amount of an active drug and optionally adding a pharmaceutically acceptable adjuvant, and carrying out uniform mixing to obtain the pharmaceutical preparation.

21. The preparation method according to claim 20, **characterized in that** the preparation method comprises the following steps: using a prescribed amount of a ligand-drug conjugate or a pharmaceutically acceptable salt thereof, a freeze-dried excipient, a pH regulator, and a solvent, dissolving each component in the solvent at once or in batches, optionally regulating the pH value to 5.0 to 8.0 by using an acidic substance or an alkaline substance in the pH regulator, and carrying out uniform mixing to obtain the pharmaceutical preparation;
preferably, when dissolving each component in the solvent, dissolving the pH regulator in the solvent at the same time or prior to the ligand-drug conjugate or the pharmaceutically acceptable salt thereof.

22. The preparation method according to claim 20 or 21, **characterized in that**
the preparation method is optionally carried out in a dark place;
and preferably, the preparation method is carried out in a dark place.

23. The preparation method according to any one of claims 20 to 22, **characterized in that**
the preparation method is carried out at a temperature of 15°C to 40°C.

24. The preparation method according to any one of claims 20 to 23, **characterized in that**
the preparation method is optionally carried out under nitrogen protection;
and preferably, the preparation method is not carried out under nitrogen protection.

25. A freeze-dried preparation, **characterized in that** the freeze-dried preparation is prepared by freeze-drying the pharmaceutical preparation according to any one of claims 1 to 19.

26. A preparation method for preparing the freeze-dried preparation according to claim 25, **characterized in that** the preparation method comprises the following steps: (i) freezing the pharmaceutical preparation at -45°C; (ii) carrying out first drying on the pharmaceutical preparation at -15°C to -5°C; and (iii) carrying out second drying on the pharmaceutical preparation at 15°C to 25°C; for example, the temperature of the first drying is -15°C, -10°C, or -5°C; the temperature of the second drying is 15°C or 25°C;
preferably, the preparation method comprises the following steps: (i) freezing the pharmaceutical preparation at -45°C; (ii) carrying out first drying on the pharmaceutical preparation at -15°C; and (iii) carrying out second drying on the pharmaceutical preparation at 25°C.

27. The freeze-dried preparation according to claim 25, **characterized in that** the freeze-dried preparation is stable at room temperature.

28. A liquid preparation, **characterized in that** the liquid preparation is reconstituted by the freeze-dried preparation according to claim 25 using water.

29. The liquid preparation according to claim 28, **characterized in that** the water comprises one or more of distilled water, pure water, and sterile water; and preferably, the freeze-dried preparation has a pH value of 5.0 to 8.0 after being reconstituted using water.

30. A preparation method for preparing the liquid preparation according to claim 28 or 29, **characterized in that** the preparation method comprises the following steps:
mixing the freeze-dried preparation with sterile water to obtain the liquid preparation.

31. A drug-containing delivery device, **characterized in that** the drug-containing delivery device comprises one of the following preparations: the pharmaceutical preparation according to any one of claims 1 to 19, the freeze-dried preparation according to claim 25, or the liquid preparation according to claim 28 or 29.

32. A pre-filled syringe, **characterized in that** the pre-filled syringe comprises one of the following preparations: the pharmaceutical preparation according to any one of claims 1 to 19, the freeze-dried preparation according to claim 25 or the liquid preparation according to claim 28 or 29, preferably for use in intravenous injection or intramuscular injection.

33. Use of the pharmaceutical preparation according to any one of claims 1 to 19, the freeze-dried preparation according to claim 25, or the liquid preparation according to claim 28 or 29 in the preparation of a delivery device or a pre-filled syringe or a drug for enhancing an immune effector cell response and/or reducing immunosuppression in a subject.

34. Use of the pharmaceutical preparation according to any one of claims 1 to 19, the freeze-dried preparation according to claim 25, or the liquid preparation according to claim 28 or 29 in the preparation of a delivery device or a pre-filled syringe or a drug for the treatment and/or prevention of a cancer, an immune disease, a cardiovascular disease, a metabolic disease and a neurological disease in a subject.

35. The use according to claim 34, **characterized in that**
the cancer comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, testicular cancer, skin cancer, lymphoma, and multiple myeloma;
the immune disease comprises one or more of a connective tissue disease, systemic sclerosis, rheumatoid arthritis, and systemic lupus erythematosus;
the cardiovascular disease comprises one or more of angina pectoris, myocardial infarction, apoplexy, heart attack, a hypertensive heart disease, a rheumatic heart disease, cardiomyopathy, cardiac arrhythmia, and a congenital heart disease;
the metabolic disease comprises one or more of diabetes mellitus, gout, obesity, hypoglycemia, hyperglycemia, and dyslipidemia;
the neurological disease comprises one or more of Alzheimer's disease, Parkinson's disease, Huntington's disease, head injury, multiple sclerosis, vertigo, coma, and epilepsy.
